(19) Europäisches Patentamt
European Patent Office
Office européen des brevets

(11) Veröffentlichungsnummer: **0 080 096 B1**

# (12) EUROPÄISCHE PATENTSCHRIFT

(45) Veröffentlichungstag der Patentschrift: **07.01.87**

(51) Int. Cl.⁴: **C 07 D 233/60**

(21) Anmeldenummer: **82110266.2**

(22) Anmeldetag: **08.11.82**

## (54) Alkylcycloalkyl-imidazolylmethyl-ketone und Verfahren zu ihrer Herstellung.

(30) Priorität: **19.11.81 DE 3145858**

(43) Veröffentlichungstag der Anmeldung: **01.06.83 Patentblatt 83/22**

(45) Bekanntmachung des Hinweises auf die Patenterteilung: **07.01.87 Patentblatt 87/2**

(84) Benannte Vertragsstaaten: **AT BE CH DE FR GB IT LI NL**

(56) Entgegenhaltungen:
**DE - A - 2 333 354**

**JOURNAL OF ORGANOMETALLIC CHEMISTRY, Band 57, 1973, M. LARCHEVEQUE et al. "Nouvelle synthèse de nitriles cyclaniques", Seiten C33-C35**

(73) Patentinhaber: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 2 (DE)**
Erfinder: **Jautelat, Manfred, Dr., Müllersbaum 28, D-5093 Burscheid (DE)**
Erfinder: **Kranz, Eckart, Dr., Am Acker 9, D-5600 Wuppertal 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Alkylcycloalkylimidazolylmethyl-ketone, ein Verfahren zu deren Herstellung und ihre Verwendung als Zwischenprodukte zur Synthese von Imidazolylvinyl-dithioacetalen, welche fungizide Eigenschaften besitzen.

Es ist bereits bekannt geworden, dass bestimmte Triazolylalkenone gute fungizide Eigenschaften aufweisen (vgl. DE-A 29 29 602). So lässt sich beispielsweise 4,4-Dimethyl-1-(naphth-2-yl)-2-(1,2,4-triazol-1-yl)-1-penten-3-on zur Bekämpfung von Pilzen einsetzen. Die Wirkung dieser Verbindung ist jedoch, insbesondere bei niedrigen Aufwandmengen und -konzentrationen, nicht immer ganz befriedigend.

Es wurden nun neue Alkylcycloalkylimidazolylmethyl-ketone der Formel

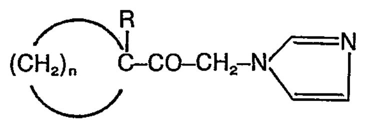

(I)

in welcher

R für geradkettiges oder verzweigtes Alkyl mit 1 bis 6 Kohlenstoffatomen steht und

n für die Zahlen 3, 4, 5, 6 und 7 steht,

gefunden.

Weiterhin wurde gefunden, dass man die neuen Alkylcycloalkyl-imidazolylmethyl-ketone der Formel (I) erhält, indem man Alkylcycloalkyl-halogenmethyl-ketone der Formel

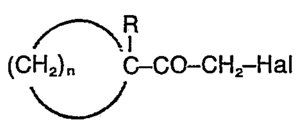

(II)

in welcher

R und n die oben angegebene Bedeutung haben und

Hal für Chlor oder Brom steht,

mit Imidazol der Formel

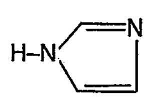

(III)

in Gegenwart eines Verdünnungsmittels und in Gegenwart eines Säurebindemittels umsetzt.

Die neuen Alkylcycloalkyl-imidazolylmethylketone sind interessante Zwischenprodukte zur Herstellung von Pflanzenschutz-Wirkstoffen. So eignen sich die Stoffe der Formel (I) z.B. als Ausgangsprodukte zur Synthese von Imidazolylvinyldithioacetalen, welche sehr gute fungizide Wirksamkeit besitzen (vgl. EP-A 0 080 103).

Überraschenderweise sind die Imidazolyl-vinyldithioacetale, die sich aus den erfindungsgemässen Alkylcycloalkylimidazolylmethyl-ketonen der Formel (I) durch aufeinanderfolgende Umsetzung mit Schwefelkohlenstoff und einem Alkylierungsmittel herstellen lassen der aus dem Stand der Technik bekannten Verbindung 4,4-Dimethyl-1-(naphth-2-yl)-2-(1,2,4-triazol-1-yl)-1-penten-3-on bezüglich ihrer fungiziden Wirksamkeit überlegen.

Die erfindungsgemässen Stoffe sind durch die Formel (I) allgemein definiert.

Besonders bevorzugt sind diejenigen Verbindungen der Formel (I), in denen R für Methyl oder Ethyl steht.

Im einzelnen seien ausser den bei den Herstellungsbeispielen genannten Verbindungen folgende Verbindungen der Formel (I) genannt:

Tabelle 1

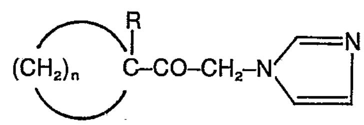

(I)

| R | n |
|---|---|
| $CH_3$ | 3 |
| $CH_3$ | 4 |
| $CH_3$ | 5 |
| $CH_3$ | 6 |
| $CH_3$ | 7 |
| $C_2H_5$ | 3 |
| $C_2H_5$ | 4 |
| $C_2H_5$ | 5 |
| $C_2H_5$ | 6 |
| $C_2H_5$ | 7 |

Verwendet man beispielsweise 1-Chloracetyl-1-ethylcyclopentan und Imidazol als Ausgangsstoffe, so kann der Reaktionsablauf bei dem erfindungsgemässen Verfahren durch das folgende Formelschema wiedergegeben werden:

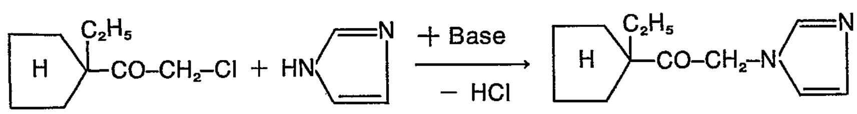


Die für die Durchführung des erfindungsgemässen Verfahrens als Ausgangsstoffe benötigten Alkylcycloalkylhalogenmethyl-ketone sind durch die Formel (II) allgemein definiert. In dieser Formel stehen R und der Index n für diejenigen Bedeutungen, die bereits im Zusammenhang mit der Beschreibung der erfindungsgemässen Stoffe der Formel (I) für den Rest R bzw. den Index n aufgeführt wurden. Hal steht für Chlor oder Brom.

Die Alkylcycloalkyl-halogenmethyl-ketone der Formel (II) sind bisher noch nicht bekannt. Sie lassen sich jedoch nach im Prinzip bekannten

Verfahren in einfacher Weise herstellen. So erhält man die Alkylcycloalkylhalogenmethyl-ketone der Formel (II), indem man 1,1-Dichloralkene der Formel

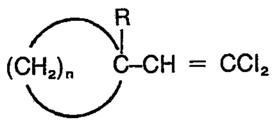 (IV)

in welcher
R und n die oben angegebene Bedeutung haben,
mit Phenolaten der Formel

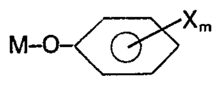 (V)

in welcher
M für ein Äquivalent eines Alkali- oder Erdalkalimetallions, insbesondere eines Natrium- oder Kaliumions steht,
X für Halogen, Alkyl oder Alkoxy mit jeweils 1 bis 3 Kohlenstoffatomen oder Phenyl steht und
m für 0, 1 oder 2 steht,
in Gegenwart eines inerten organischen Lösungsmittels, wie z.B. Dimethylformamid, bei Temperaturen zwischen 100 und 220 °C, gegebenenfalls unter erhöhtem Druck, umsetzt und die dabei erhaltenen Phenylether der Formel

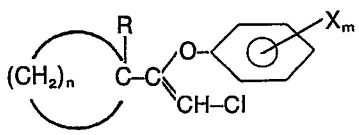 (VI)

in welcher
R, X, m und n die oben angegebene Bedeutung haben
in üblicher Weise mit Mineralsäuren, wie z.B. Schwefelsäure oder Salzsäure, und/oder mit organischen Säuren, wie z.B. Ameisensäure, bei 40 bis 100 °C hydrolysiert.

Die Herstellung von 1,1-Dichloralkenen der Formel (IV) ist bekannt. Sie erfolgt durch Addition von Alkylhalogeniden an Vinylidenchlorid in Gegenwart von sauren Katalysatoren (vgl. hierzu J. Amer. Chem. Soc. 74, 2885 (1952)) mit gleichzeitiger Abspaltung von Halogenwasserstoff.

Die Phenolate der Formel (V) sind allgemein bekannte Verbindungen der organischen Chemie.

Die Alkylcycloalkyl-halogenmethyl-ketone der Formel (II) können auch erhalten werden, indem man Alkylcycloalkylmethyl-ketone der Formel

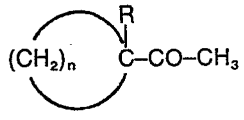 (VII)

in welcher
R und n die oben angegebene Bedeutung haben,
in üblicher Weise mit Chlor oder Brom in Gegenwart eines inerten organischen Lösungsmittels, wie beispielsweise Ether, chlorierten oder nichtchlorierten Kohlenwasserstoffen, bei Raumtemperatur umsetzt, oder mit üblichen Chlorierungsmitteln, wie beispielsweise Sulfurylchlorid, bei 20 bis 60 °C umsetzt.

Die Alkylcycloalkyl-methyl-ketone der Formel (VII) werden erhalten, indem man entsprechende Nitrile der Formel

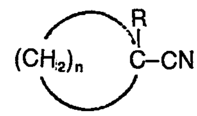 (VIII)

in welcher
R und n die oben angegebene Bedeutung haben,
in üblicher Weise mit metallorganischen Verbindungen, wie insbesondere Methylmagnesiumbromid, in Gegenwart eines Verdünnungsmittels, wie z.B. eines wasserfreien Ethers, bei Temperaturen zwischen 0 und 80 °C umsetzt.

Die Nitrile der Formel (VIII) sind bekannt (vgl. Journal of Organometallic Chemistry 57, C 33–35 (1973)) bzw. können sie nach dem dort angegebenen Verfahren erhalten werden.

Für das erfindungsgemässe Verfahren kommen als Verdünnungsmittel inerte organische Lösungsmittel in Frage. Hierzu gehören vorzugsweise Ketone, wie Aceton, Methylethylketon und Methylbutylketon; Alkohole, wie Ethanol, Isopropanol und Butanol; aromatische Kohlenwasserstoffe, wie Benzol und Toluol; Formamide und Sulfoxide, wie Dimethylformamid und Dimethylsulfoxid; sowie Nitrile, wie Acetonitril.

Das erfindungsgemässe Verfahren wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, wie Alkali- und Erdalkalihydroxide, beispielsweise Kaliumhydroxid und Calciumhydroxid, oder wie niedere tertiäre Alkylamine, Cycloalkylamine oder Aralkylamine, beispielsweise Triethylamin, N,N-Dimethylmethylcyclohexylamin, Dicyclohexylamin, N,N-Dimethylbenzylamin, weiterhin Pyridin und Diazabicyclooctan.

Vorzugsweise verwendet man einen entsprechenden Überschuss an Imidazol.

Die Reaktionstemperaturen können bei dem erfindungsgemässen Verfahren in einem grösseren Bereich variiert werden. Im allgemeinen arbeitet man bei Temperaturen zwischen 0 und 120 °C, vorzugsweise zwischen 20 und 90 °C. Zweckmässigerweise arbeitet man beim Siedepunkt des jeweiligen Lösungsmittels.

Bei der Durchführung des erfindungsgemässen Verfahrens setzt man auf 1 Mol der Verbindungen der Formel (II) vorzugsweise 1 bis 4 Mol Imidazol und 1 bis 4 Mol Säurebinder ein. Zur Isolierung der Verbindungen der Formel (I) wird das Lösungsmittel abdestilliert und der Rückstand in üblicher Art und Weise aufgearbeitet.

Die erfindungsgemässen Alkylcycloalkyl-imidazolylmethylketone der Formel (I) eignen sich als Zwischenprodukte zur Synthese von Imidazolyl-vinyl-dithioacetalen, welche fungizide Wirksamkeit besitzen (vgl. EP-A 0 080 103).

Solche Imidazolyl-vinyl-dithioacetale der Formel

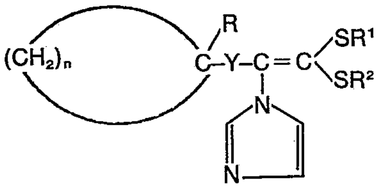

(IX)

in welcher

R und n die oben angegebene Bedeutung haben,

$R^1$ und $R^2$ gleich sind und für Alkyl, Alkenyl, Alkinyl, gegebenenfalls substituiertes Benzyl oder Trialkylsilyl stehen, oder

$R^1$ und $R^2$ gemeinsam für eine Alkylenkette, für eine Dialkylsilylbrücke oder die $-CH=CH$-Gruppe stehen und

Y für eine Ketogruppe oder eine CH(OH)-Gruppe steht,

lassen sich herstellen, indem man Alkylcycloalkyl-imidazolylmethyl-ketone der Formel

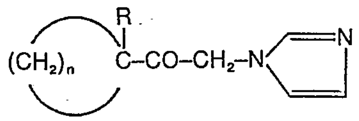

(I)

in welcher

R und n die oben angegebene Bedeutung haben,

zunächst mit Schwefelkohlenstoff in Gegenwart einer Base und in Gegenwart eines Verdünnungsmittels versetzt und anschliessend wieder

α) mit einer Verbindung der Formel

$$Hal'\text{-}R^3 \qquad (X)$$

in welcher

Hal' für Halogen steht und

$R^3$ für Alkyl, Alkenyl, Alkinyl, gegebenenfalls substituiertes Benzyl oder Trialkylsilyl steht, oder

β) mit Dimethylsulfat der Formel

$$(CH_3)_2SO_4 \qquad (XI)$$

oder

γ) mit einem Dihalogenid der Formel

$$Hal''\text{-}R^4\text{-}Hal'' \qquad (XII)$$

in welcher

Hal'' für Halogen steht und

$R^4$ für eine ein- oder mehrgliedrige Methylenkette, für eine Dialkylsilylbrücke oder eine $-CH=CH$-Gruppe steht,

in Gegenwart des gleichen Verdünnungsmittels umsetzt; und gegebenenfalls die erhaltenen Keto-Derivate der Formel

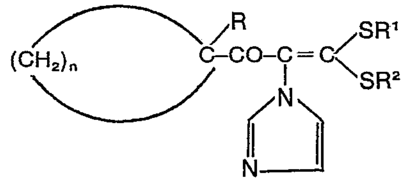

(IXa)

in welcher

R, $R^1$, $R^2$ und n die oben angegebene Bedeutung haben,

in allgemein üblicher Weise reduziert.

Die aus den erfindungsgemässen Stoffen herstellbaren Imidazolyl-vinyl-dithioacetale sind durch die Formel (IX) allgemein definiert. In dieser Formel stehen R und der Index n für diejenigen Bedeutungen, die bereits im Zusammenhang mit der Beschreibung der erfindungsgemässen Stoffe der Formel (I) für den Rest R und den Index n genannt wurden. Ausserdem stehen vorzugsweise $R^1$ und $R^2$ für den gleichen Rest, wie geradkettiges oder verzweigtes Alkyl mit 1 bis 4 Kohlenstoffatomen, geradkettiges oder verzweigtes Alkenyl und Alkinyl mit jeweils 2 bis 6 Kohlenstoffatomen, gegebenenfalls substituiertes Benzyl, wobei als Substituenten am Phenylring der Benzylgruppe in Frage kommen: Halogen, Alkyl, Alkoxy und Alkylthio mit jeweils 1 bis 4 Kohlenstoffatomen; Halogenalkyl, Halogenalkoxy und Halogenalkylthio mit jeweils 1 bis 2 Kohlenstoff- und bis zu 5 gleichen oder verschiedenen Halogenatomen, wie insbesondere Fluor- und Chloratomen, sowie gegebenenfalls durch Halogen und/oder Alkyl mit 1 bis 2 Kohlenstoffatomen substituiertes Phenyl und Phenoxy; und weiterhin stehen vorzugsweise

$R^1$ und $R^2$ jeweils für Trialkylsilyl mit 1 bis 4 Kohlenstoffatomen in jedem Alkylteil;

$R^1$ und $R^2$ gemeinsam für eine Alkylenkette mit 1 bis 4 Kohlenstoffatomen, für eine Dialkylsilylbrücke mit 1 bis 4 Kohlenstoffatomen in jedem Alkylteil oder für die $-CH=CH$-Gruppe und

Y für die Ketogruppe oder die CH(OH)-Gruppe.

Die bei der Herstellung der Imidazolyl-vinyl-dithioacetale als Reaktionskomponenten zu verwendenden Verbindungen sind durch die Formeln (X), (XI) und (XII) definiert. In der Formel (X) steht Hal' vorzugsweise für Chlor oder Brom, und $R^3$ steht vorzugsweise für die Bedeutungen, die bei der Beschreibung der Stoffe der Formel (IX) bereits vorzugsweise für $R^1$ genannt wurden. In der Formel (XII) steht Hal'' vorzugsweise für Chlor oder Brom, und $R^4$ steht vorzugsweise für die Bedeutungen, die bei der Beschreibung der Stoffe der Formel (IX) bereits vorzugsweise für die gemeinsame Substituentenbildung von $R^1$ und $R^2$ genannt wurden.

Die Verbindungen der Formeln (X), (XI) und (XII) sind allgemein bekannte Verbindungen der organischen Chemie.

Als Verdünnungsmittel kommen für das Verfahren zur Herstellung der Keto-Derivate der Formel (IXa) unter den Reaktionsbedingungen inerte organische Lösungsmittel in Frage. Hierzu gehören vorzugsweise Ether, wie Tetrahydrofuran oder Dioxan; Alkohole, wie Methanol, Ethanol oder Isopropanol; Amide, wie Dimethylformamid oder Dimethylacetamid; ferner Dimethylsulfoxid, Hexamethylphosphorsäuretriamid oder Sulfolan (Tetrahydrothiophen-1,1-dioxid).

Das Verfahren zur Herstellung der Keto-Derivate der Formel (IXa) wird in Gegenwart einer Base durchgeführt.

Hierzu gehören vorzugsweise Alkalihydroxide und -alkoholate, wie Natrium- und Kaliumhydroxid oder Natrium- und Kalium-methylat, -ethylat und -tert.-butylat; Alkalihydroxide und -amide, wie Natriumhydrid, Natriumamid oder Lithiumisopropylamid, sowie Butyllithium.

Die Reaktionstemperaturen können bei der Durchführung des Verfahrens zur Herstellung der Keto-Derivate der Formel (IXa) in einem grösseren Bereich variiert werden. Im allgemeinen arbeitet man zwischen 0 und 80°, vorzugsweise zwischen 0 und 60 °C.

Bei der Durchführung des Verfahrens zur Herstellung der Keto-Derivate der Formel (IXa) setzt man auf 1 Mol Alkylcycloalkyl-imidazolylmethylketon vorzugsweise 1 bis 1,1 Mol Schwefelkohlenstoff und 2 Mol Base sowie 2 Mol einer Verbindung der Formel (XII) ein. Dabei wird vorzugsweise das Keton der Formel (I) vorgelegt, zunächst mit der halben Menge Schwefelkohlenstoff und Base versetzt, anschliessend mit der restlichen Menge Schwefelkohlenstoff und Base und zum Schluss mit der jeweiligen Reaktionskomponente der Formel (X), (XI) oder (XII). Die Isolierung der Verbindung der Formel (IXa) erfolgt in üblicher Weise.

Arbeitet man bei der Reduktion der Keto-Derivate der Formel (IXa) mit Aluminiumisopropylat, so kommen als Verdünnungsmittel für diese Umsetzung bevorzugt Alkohole, wie Isopropanol, oder inerte Kohlenwasserstoffe, wie Benzol, in Frage. Die Reaktionstemperaturen können wiederum in einem grösseren Bereich variiert werden; im allgemeinen arbeitet man zwischen 20 und 120 °C, vorzugsweise zwischen 50 und 100 °C. Zur Durchführung der Reaktion setzt man auf 1 Mol eines Ketons der Formel (IXa) etwa 1 bis 2 Mol Aluminiumisopropylat ein. Zur Isolierung der reduzierten Verbindungen der Formel (IX) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Die aus den erfindungsgemässen Stoffen der Formel (I) herstellbaren Imidazolyl-vinyl-dithioacetale der Formel (IX) besitzen sehr gute fungizide Eigenschaften (vgl. EP-A 0 080 103).

## Herstellungsbeispiele

### Beispiel 1

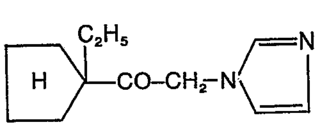

100 g (0,57 Mol) 1-Chlor-acetyl-1-ethylcyclopentan und 137 g Imidazol werden in 1000 ml Acetonitril 48 Stunden unter Rückfluss erhitzt. Danach lässt man abkühlen und engt das Reaktionsgemisch ein. Der Rückstand wird in 1000 ml Wasser aufgenommen und mit je 500 ml Methylenchlorid zweimal ausgeschüttelt. Die vereinigten organischen Phasen werden dreimal mit je 200 ml Wasser gewaschen, über Natriumsulfat getrocknet und eingeengt. Man erhält 95 g (76% der Theorie) 1-Ethylcyclopentyl-(imidazol-1-yl)-methyl-keton in Form eines viskosen Öles.

Herstellung des Ausgangsproduktes

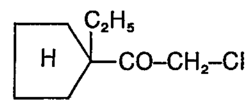

(II-1)

125,3 g (0,5 Mol) 1-Chlor-2-(1-ethylcyclopentyl)-2-phenoxyethylen werden in 500 ml Ameisensäure und 50 ml konzentrierter Salzsäure 2 Stunden auf 80 °C erwärmt. Dann wird mit Methylenchlorid und Eis verdünnt und dreimal mit 2n Natronlauge ausgeschüttelt. Nach dem Trocknen der Methylenchloridphase über Natriumsulfat wird das Lösungsmittel im Vakuum abrotiert. Der Rückstand wird im Vakuum destilliert. Man erhält 72 g (82,6% der Theorie) 1-Chloracetyl-1-ethylcyclopentan vom Brechungsindex $n_D^{20}$ = 1,484.

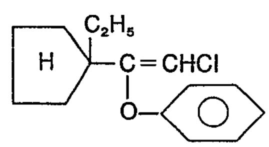

(VI-1)

140 g (4,2 Mol) Natriumphenolat werden in 500 ml N-Methylpyrrolidon auf 200 °C erhitzt. 116 g (0,6 Mol) 1-(2,2-Dichlorvinyl)-1-ethyl-cyclopentan werden so langsam zugetropft, dass die Reaktionstemperatur nicht unter 195 °C sinkt. Dann wird 1 Stunde bei 210 °C nachgerührt. Nach dem Abkühlen wird mit Methylenchlorid verdünnt und mit 2n Natronlauge mehrfach ausgeschüttelt. Die über Natriumsulfat getrocknete organische Phase wird im Vakuum eingeengt, und der Rückstand wird fraktioniert. Man erhält 132 g (88% der Theorie) 1-Chlor-2-(1-ethylcyclopentyl)-2-phenoxyethylen vom Siedepunkt 115–125 °C/0,1 Torr.

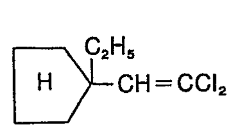

(IV-1)

Zu 291 g (3 Mol) 1,1-Dichlorethen werden bei −20 °C 10 g wasserfreies Aluminiumchlorid gegeben und danach bei 0–10 °C 133 g (1 Mol) 1-Ethylcyclopentylchlorid (Chem. Abstr. 42, 6328 (1948)) eingetropft. Man lässt die Reaktionslösung auf 20 °C erwärmen, setzt weitere 5 g Aluminiumchlorid zu und rührt 2 Stunden bei 20 °C nach. Der Ansatz wird auf Eis gegossen, mit Methylenchlorid und verdünnter Salzsäure aufgearbeitet. Aus der organischen Phase gewinnt man nach Trocknen über Natriumsulfat durch fraktionierte Destillation 158 g (82% der Theorie) 1-(2,2-Dichlorvinyl)-1-ethyl-cyclopentan vom Siedepunkt 45–50 °C/0,1 Torr.

In analoger Weise und entsprechend dem erfindungsgemässen Verfahren werden die in der Tabelle 2 aufgeführten Verbindungen der Formel

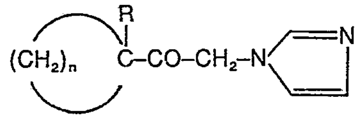
(I)

erhalten:

Tabelle 2

| Beispiel Nr. | n | R | Schmelzpunkt (°C) bzw. Brechungsindex $n_D^{20}$ |
|---|---|---|---|
| 2 | 5 | $CH_3$ | 75–76 |

Entsprechend Beispiel 1 und gemäss den angegebenen Verfahren werden die in der Tabelle 3 aufgeführten Ausgangsstoffe der Formel

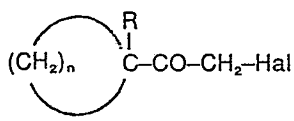
(II)

erhalten:

Tabelle 3

| Beispiel Nr. | n | R | Hal | Physikal. Konstante Kp (°C) / mbar |
|---|---|---|---|---|
| (II–2) | 5 | $CH_3$ | Cl | 125–130 / 18 |
| (II–3) | 4 | $C_4H_9$ | Cl | 82– 88 / 0,1 |
| (II–4) | 6 | $CH_3$ | Cl | 75– 78 / 0,1 |
| (II–5) | 7 | $CH_3$ | Cl | 92– 96 / 0,03 |

**Patentansprüche**

1. Alkylcycloalkyl-imidazolylmethyl-ketone der Formel

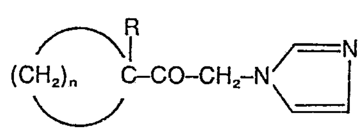
(I)

in welcher

R für geradkettiges oder verzweigtes Alkyl mit 1 bis 6 Kohlenstoffatomen steht und

n für die Zahlen 3, 4, 5, 6 und 7 steht.

2. Verfahren zur Herstellung von Alkylcycloalkyl-imidazolylmethyl-ketonen der Formel

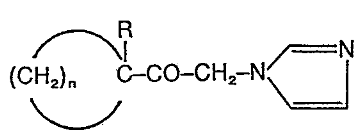
(I)

in welcher

R für geradkettiges oder verzweigtes Alkyl mit 1 bis 6 Kohlenstoffatomen steht und

n für die Zahlen 3, 4, 5, 6 und 7 steht,

dadurch gekennzeichnet, dass man Alkylcycloalkyl-halogenmethyl-ketone der Formel

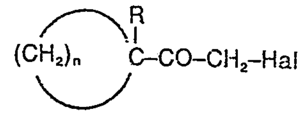
(II)

in welcher

R und n die oben angegebene Bedeutung haben und

Hal für Chlor oder Brom steht,

mit Imidazol der Formel

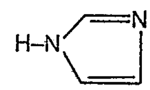
(III)

in Gegenwart eines Verdünnungsmittels und in Gegenwart eines Säurebindemittels umsetzt.

**Claims**

1. Alkylcycloalkyl imidazolylmethyl ketones of the formula

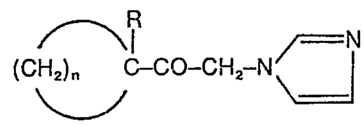
(I)

in which

R represents straight-chain or branched alkyl with 1 to 6 carbon atoms and

n represents the numbers 3, 4, 5, 6 and 7.

2. Process for the preparation of alkylcycloalkyl imidazolylmethyl ketones of the formula

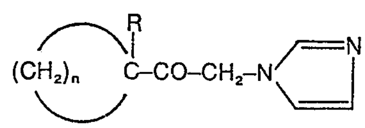
(I)

in which

R represents straight-chain or branched alkyl with 1 to 6 carbon atoms and

n represents the numbers 3, 4, 5, 6 and 7, characterised in that alkylcycloalkyl halogenomethyl ketones of the formula

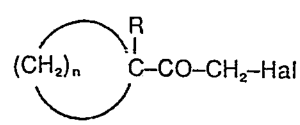
(II)

in which

R and n have the abovementioned meaning and

Hal represents chlorine or bromine,

are reacted with imidazole of the formula

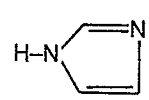
(III)

in the presence of a diluent and in the presence of an acid-binding agent.

**Revendications**

1. Alkylcycloalkyl-imidazolylméthyl-cétones de formule

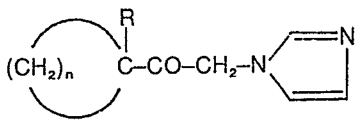

(I)

dans laquelle

R représente un groupe alkyle à chaîne droite ou ramifiée en $C_1$–$C_6$, et

n est égal à 3, 4, 5, 6 ou 7.

2. Procédé de préparation des alkylcycloalkyl-imidazolylméthyl-cétones de formule

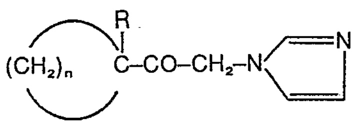

(I)

dans laquelle

R représente un groupe alkyle à chaîne droite ou ramifiée en $C_1$–$C_6$, et

n est égal à 3, 4, 5, 6 ou 7,

caractérisé en ce que l'on fait réagir des alkylcycloalkyl-halogénométhyl-cétones de formule

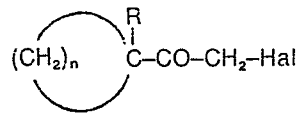

(II)

dans laquelle

R et n ont les significations indiquées ci-dessus et

Hal représente le chlore ou le brome,

avec l'imidazole de formule

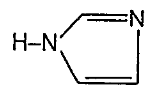

(III)

en présence d'un diluant et en présence d'un agent captant les acides.